# EUROPÄISCHE PATENTSCHRIFT

(11) **EP 1 898 823 B1**
(45) Veröffentlichungstag und Bekanntmachung des Hinweises auf die Patenterteilung: **02.09.2015**
(21) Anmeldenummer: 06743378.9
(22) Anmeldetag: 21.04.2006
(51) Int. Cl.: A61B 18/14, A61B 18/00

(54) **BIEGEWEICHE APPLIKATIONSVORRICHTUNG ZUR HOCHFREQUENZTHERAPIE VON BIOLOGISCHEM GEWEBE**
FLEXIBLE APPLICATION DEVICE FOR THE HIGH-FREQUENCY TREATMENT OF BIOLOGICAL TISSUE
DISPOSITIF D'APPLICATION FLEXIBLE POUR REALISER UNE THERAPIE HAUTE FREQUENCE SUR UN TISSU BIOLOGIQUE

(30) Priorität: 13.05.2005 DE 102005023303
(43) Veröffentlichungstag der Anmeldung: 19.03.2008
(73) Patentinhaber: Olympus Winter & Ibe GmbH, 22045 Hamburg (DE)
(72) Erfinder: DESINGER, Kai, 14195 Berlin (DE); ROGGAN, André, 12163 Berlin (DE); STEIN, Thomas, 14513 Teltow (DE); FAY, Markus, 14513 Teltow (DE)
(74) Vertreter: Eisenführ Speiser
(86) Internationale Anmeldenummer: PCT/EP2006/061762
(87) Internationale Veröffentlichungsnummer: WO 2006/120116

(56) Entgegenhaltungen:
- WO-A-00/36985
- WO-A-2006/003216
- DE-A1- 4 442 690
- DE-A1- 10 128 701
- US-A1- 2003 088 242
- US-B1- 6 514 251

## Beschreibung

Die Erfindung betrifft eine biegeweiche Applikationsvorrichtung zur Hochfrequenztherapie von biologischem Gewebe. Die Applikationsvorrichtung umfasst einen röhrenförmigen Hochfrequenzkatheter mit einem vorzugsweise biegeweichen, zumindest ein durchgehendes Lumen aufweisenden Schaftrohr, der an oder in einem Verbindungselement ansetzt, und mindestens einer am distalen Ende des Hochfrequenzkatheters angeordneten Kopfelektrode.

Insbesondere betrifft die Erfindung eine Applikationsvorrichtung für den endoluminalen oder interstitiell-endoskopischen Einsatz.

Applikationsvorrichtungen zur Hochfrequenztherapie sind bekannt. Während einer solchen Therapie wird durch Anlegen einer hochfrequenten Wechselspannung zwischen zwei Elektroden eine thermische Tiefenerwärmung des die Elektroden umgebenden Gewebes erreicht. Die sogenannten aktiven Bereiche der Elektroden stehen nach dem Einführen in den Körper des Patienten mit dem Körpergewebe in elektrisch leitfähiger Verbindung. Durch den ohmschen Gewebewiderstand, der ein Teil der komplexen Gewebeimpedanz ist, erfolgt eine Umsetzung des über die Elektroden applizierten Wechselstroms in joulesche Wärme. Bei Temperaturen zwischen 50°C und 100°C kommt es zu einer Denaturierung der körpereigenen Proteine (Koagulation) und in der Folge zum Schrumpfen bzw. Absterben der betroffenen Gewebsareale. Auf Grund der hohen Stromdichte an den aktiven Elektroden erfolgt die Erwärmung vorwiegend im Bereich dieser Elektroden, so dass eine lokale thermische Applikation möglich ist.

Aus US 6,014,589, US 6,036,687, US 6,071,277, DE 101 28 701, WO 00/36985, DE 44 42 690, US 2003/0088242 und US 2004/0162555 A1 sind Verfahren und verschiedene Vorrichtungen zur Hochfrequenztherapie von Hohlorganen, insbesondere zur Behandlung von Venen bekannt.

Applikationsvorrichtungen zur Hochfrequenztherapie von oder in Hohlorganen der gattungsgemäßen Art umfassen einen röhrenförmigen Hochfrequenzkatheter mit
- einem biegeweichen, zumindest ein durchgehendes Lumen aufweisenden Schaftrohr,
- einer am distalen Ende des Hochfrequenzkatheter angeordneten Kopfelektrode,
- einer elektrischen Leitung mit einem Anschluss für einen Hochfrequenzgenerator, und
- einem Verbindungselement zwischen dem Schaftrohr und der Leitung.

Der vorliegenden Erfindung liegt nun die Aufgabe zugrunde, eine Applikationsvorrichtung der gattungsgemäßen Art bereitzustellen, die eine größere Anwendungssicherheit bietet.

Erfindungsgemäß wird diese Aufgabe durch eine biegeweiche Applikationsvorrichtung zur Hochfrequenztherapie von biologischem Gewebe gemäß Anspruch 1 gelöst, die ein sich zwischen der Kopfelektrode und dem Verbindungselement erstreckendes Zugelement aufweist, welches mit der Kopfelektrode einerseits und dem Verbindungselement andererseits derart fest verbunden ist und dessen Querschnittsfläche und Zugfestigkeit so bemessen ist, dass sämtliche während einer Behandlung auftretenden äußeren, auf den Hochfrequenzkatheter einwirkenden Kräfte über die Kopfelektrode und das Zugelement in das Verbindungselement übertragen werden können.

Der Erfindung liegt die Erkenntnis zugrunde, dass bei herkömmlichen Applikationsvorrichtungen, die während einer Behandlung auftretenden äußeren, auf den Hochfrequenzkatheter einwirkenden Kräfte im wesentlichen über äußere Bestandteile der Applikationsvorrichtung in das Verbindungselement übertragen werden. Die äußeren Bestandteile der Applikationsvorrichtung, zu denen üblicherweise eine flüssigkeitsdicht, elektrisch isolierende Hülle zählt, sind häufig in Längsrichtung des Hochfrequenzkatheters mehrstückig, so dass sich in Längsrichtung des Hochfrequenzkatheters mehrere Verbindungsstellen ergeben, über die die äußeren Kräfte zu übertragen sind. Jede der Verbindungsstellen stellt jedoch einen potentiellen Schwachpunkt dar. Hierdurch besteht die Gefahr eines Lösens oder gar Abreißens einzelner Bestandteile des Hochfrequenzkatheters bei mechanischer Belastung. Dieses Problem wird durch ein von der Kopfelektrode zum Verbindungselement durchgehendes Zugelement, das an der Kopfelektrode einerseits und an dem Verbindungselement andererseits entsprechend fest befestigt ist, wirkungsvoll verhindert.

Das Schaftrohr des Hochfrequenzkatheters bildet vorzugsweise eine elektrisch isolierende Hülle, die das Lumen einschließt oder ist mit einer solchen Hülle versehen.

Die Kopfelektrode ist über eine elektrisch leitende Zuleitung, die innerhalb der elektrisch isolierenden Hülle verläuft, mit einer elektrischen Kontaktstelle in dem Verbindungselement elektrisch verbunden. Diese Zuleitung bildet das Zugelement selbst und hat somit eine Doppelfunktion elektrischer Leiter und Sicherungselement. Bei geeigneter Wahl des Zuleitungsmaterials und geeigneter Dimensionierung können die mechanischen Anforderungen an das Zugelement und die elektrischen Anforderungen an die Zuleitung gleichermaßen erfüllt werden.

Die Zuleitung als Zugelement besitzt vorzugsweise eine Zugfestigkeit von mindestens 1000 N/mm² und einen Durchmesser zwischen 0,2 und 0,8 mm, besonders bevorzugt 0,4 mm. Ein derartiges Zugelement kann bei einem Durchmesser von 0,4 mm Zugkräfte von mehr als 125 N von der Kopfelektrode zum Verbindungselement übertragen. Diese Zugkraft reicht aus, um bei Versagen sämtlicher Verbindungsstellen zwischen der Hülle des Schaftrohres und den Elektroden sowie zwischen den potentiell mehreren Elektroden und den jeweiligen Isolatorelementen alle äußeren Kräfte von der Kopfelektrode in das Verbindungselement zu übertragen. Das Zugelement funktioniert dabei als Sicherheitselement. Die auf den Hochfrequenzkatheter einwirkenden äußeren Kräfte beim Herausziehen aus dem Gewebe sind üblicherweise in Längsrichtung des Hochfrequenzkatheters gerichtet. Die Kopfelektrode fängt diese Kräfte auf und leitet sie über das Zugelement als Zugkräfte in das Verbindungselement ein. Bei Versagen aller Verbindungsstellen der äußeren Bestandteile des Hochfrequenzkatheters sind diese äußeren Bestandteile immer noch wie auf einer Perlenschnur auf die als Zugelement dienende Zuleitung aufgezogen und auf diese Weise durch die Zuleitung miteinander verbunden. Ein Abrutschen der äußeren Bestandteile des Hochfrequenzkatheters wird dadurch verhindert, dass die Kopfelektrode einen Durchmesser aufweist, der dies verhindert.

Die maximale Zugkraft des Zugelements und die Verbindung zu der Kopfelektrode liegt im Bereich von 70 bis 300 N.

Die Angaben zur Zugfestigkeit beziehen sich im Rahmen dieser Beschreibung auf den Maximalwert der Zugspannung für ein jeweiliges Material, der sich als Maximum Rₘ aus einer Spannungs-Dehnungs-Kurve ergibt, die im Zugversuch ermittelt wird. Die Zugfestigkeit errechnet sich aus dem Quotient einer maximalen Zugkraft und dem Ausgangsquerschnitt der Probe (Maßeinheit: N/mm²).

Der erwähnte, bevorzugte Durchmesser der Zuleitung der Kopfelektrode im Bereich von 0,2 - 0,8 mm ermöglicht neben einer ausreichenden maximalen Zugkraft und einer guten elektrischen Leitfähigkeit auch eine einfache Integration des Hochfrequenzkatheters in bestehende Applikationsvorrichtungen. Weiterhin gewähren Leitungen dieses Durchmessers eine für die endoluminale bzw. interstitiell-endoskopische Anwendung hinreichende Flexibilität.

Ein besonders geeignetes Material für die Zuleitung als Zugelement ist ein nichtrostendes Metall wie Titan oder nichtrostender Stahl. Diese Metalle besitzen eine geeignet hohe Zugfestigkeit und bieten gleichzeitig aufgrund der Dimensionierung des Durchmessers der Zuleitung auch eine ausreichende elektrische Leitfähigkeit.

In einer besonders bevorzugten Ausführungsvariante besteht die Zuleitung der Kopfelektrode ganz oder in Teilen aus nichtrostendem Stahl, der auch als V2A-Stahl bezeichnet wird. Der Werkstoff besitzt eine hohe Zugfestigkeit und ist biokompatibel. Die Zuleitung kann in einer alternativen Ausführungsform ein geflochtener Draht aus einer Vielzahl von einzelnen Filamenten aus rostfreiem Stahl sein. Hierdurch ist die für die Applikation notwendige Flexibilität des Hochfrequenzkatheters - insbesondere bei den vorgenannten bevorzugten Durchmessern der Zuleitung - noch sichergestellt. Besonders bevorzugt ist die Wahl des Werkstoffs Stahl immer dann, wenn auch die Kopfelektrode zumindest in einem Anschlussbereich der Zuleitung aus Stahl besteht. In diesem Fall kann durch Verschweißen eine homogene und hochfeste Verbindung zwischen der Zuleitung und der Kopfelektrode geschaffen werden, die auch hohen mechanischen Belastungen standhält.

Das Koagulationsvolumen von Applikationsvorrichtungen ist dadurch begrenzt, dass das Gewebe in Elektrodennähe, wo Temperaturen von über 100 C auftreten, nach einer gewissen Applikationszeit durch Verdampfen der Gewebeflüssigkeit austrocknet. Dieses Austrocknen bedingt einen Anstieg des spezifischen Widerstandes des Gewebes. Ist mindestens eine Elektrode vollkommen von ausgetrocknetem Gewebe umschlossen, kommt es zu einem rapiden Anstieg der Abschlußimpedanz, so dass ein weiterer Energieeintrag in das Gewebe verhindert wird. Dies ist gleichbedeutend mit einem Abbruch der Applikation, auch wenn der Hochfrequenzgenerator eingeschaltet bleibt.

Durch die interne Spülung des Hochfrequenzkatheters mit einem temperierten Medium (z. B. Wasser) kann dieser Vorgang in gewissen Grenzen vermieden werden. Mit Hilfe eines internen Flüssigkeitskreislaufs werden die Elektroden permanent auf die Temperatur der durchlaufenden Flüssigkeit gehalten. Bei Wahl einer Flüssigkeitstemperatur von Raumtemperatur kann schon vermieden werden, dass sich das Gewebe in Elektrodennähe so stark erwärmt, dass es austrocknet. Die Regionen mit der höchsten Temperatur, die sich bei einem ungespülten Hochfrequenzkatheter direkt an der Elektrodenoberfläche befinden, verlagern sich in tiefere Gewebeschichten. Das Gewebe in Elektrodennähe behält seinen Wasser- und Elektrolytgehalt und verliert so nicht an elektrischer Leitfähigkeit. Dies hat die Konsequenz, dass dort selbst nach langen Applikationszeiten und bei hohen Leistungen elektrische Energie in Wärme transformiert werden kann.

Die Elektrodentemperierung basiert auf einem Gegenstromflüssigkeitskreislauf. In einer bevorzugten Ausführungsvariante ist das Zugelement als ein Rohr ausgeführt, in dem die Flüssigkeit bis an die Spitze des Hochfrequenzkatheters geführt wird und an den Elektroden entlang wieder zurück zum proximalen Ende des Hochfrequenzkatheters zurückströmt. Die Flüssigkeitsmenge, die durch den Hochfrequenzkatheter gepumpt wird liegt zwischen 10 und 100 ml pro Minute.

Eine weitere Möglichkeit, die Unterbrechung des elektrischen Stromes durch einen Impedanzanstieg infolge von Gewebedehydrierung zu vermeiden, ist eine offene Spülung. Bei diesem Prinzip wird vorzugsweise elektrisch leitende Flüssigkeit (z. B. physiologische Kochsalzlösung) durch kleine Bohrungen oder Schlitze am distalen Ende des Hochfrequenzkatheters (z. B. in den Elektroden oder in dem bei einer bipolaren Anordnung zwischen den Elektroden liegenden Isolator) in das Gewebe gepumpt. Hierbei werden vorzugsweise nur sehr geringe Mengen Flüssigkeit in das Gewebe abgegeben und am besten nur dann, wenn die Dehydrierung einsetzt oder vorliegt. Die Steuerung der Pumpe kann zu diesem Zweck impedanzabhängig erfolgen: Wird ein Impedanzanstieg detektiert, was mit einer elektrodennahen Gewebedehydrierung gleichzusetzen ist, dann fördert die Pumpe Flüssigkeit bis die Impedanz wieder auf einen normalen Wert kommt. Die Flüssigkeitsmengen, die hier bei einem Pumpvorgang gefördert werden sind um ein vielfaches geringer als beim oben beschriebenen geschlossen Flüssigkeitskreislauf und liegt beispielsweise zwischen 10 und 200 ml pro Stunde. Nicht nur durch Variation der Spülmenge, sondern auch der Salzkonzentration der Lösung kann die erzielbare Steigerung der elektrischen Leitfähigkeit des Gewebes an den Bedarf angepasst werden.

Für das offene Spülprinzip können noch andere Arten von Flüssigkeiten zum Einsatz kommen.

### Therapeutisch wirksame Flüssigkeit:

Durch kleine Bohrungen oder Schlitze am distalen Ende des Hochfrequenzkatheters kann ein therapeutisch wirksames Medikament direkt in den Behandlungsort injiziert werden. Denkbar ist hier z. B. ein Chemotherapeutikum, dass die mittels Hochfrequenzstrom erwärmten Tumorzellen besonders gut zerstört oder zu einer Wärmesensibilisierung der Tumorzellen führt und so die Effektivität der Thermotherapie steigert. Möglich ist aber auch die Injektion eines Lokalanästhetikums in das zu behandelnde Gewebe, um die durch die Gewebeerwärmung nach dem Einschalten des Hochfrequenzstromes auftretenden Schmerzentwicklungen zu reduzieren

### Toxische Flüssigkeit:

Es ist bekannt, dass durch die Injektion von hochkonzentriertem Alkohol eine lokale Zerstörung von Tumorzellen erreicht werden kann. Die Injektion kann im Falle der Erfindung durch kleine Bohrungen oder Schlitze am distalen Ende des Hochfrequenzkatheters erfolgen und die Thermotherapie ergänzen. Die Abgabe einer toxischen Substanz wie (z. B. hochprozentiger Alkohol oder eine hochkonzentrierte Salzlösung) kann auch nach der Thermotherapie während des Herausziehens des Hochfrequenzkatheters aus dem Gewebe erfolgen, um die Streuung von Tumorzellen zu vermeiden.

### Mischung:

Letztendlich ist auch eine Mischung aus elektrisch leitender, therapeutisch wirksamer und toxischer Flüssigkeiten möglich, die an die jeweilige Behandlung angepasst und optimiert werden kann.

Vorzugsweise ist die Applikationsvorrichtung als bipolare Applikationsvorrichtung mit einer zweiten, gegenüber der Kopfelektrode elektrisch isolierten proximalen Elektrode ausgestattet. Die proximale Elektrode ist in der Nähe des distalen Endes des Hochfrequenzkatheters angeordnet und über eine zweite elektrische Zuleitung mit einer zweiten Kontaktstelle in dem Verbindungselement elektrisch leitend verbunden. Der Abstand zwischen der Kopfelektrode und der proximalen Elektrode beträgt vorzugsweise zwischen 5 und 20 % der Elektrodenlänge (bei bipolarer Ausführung entspricht dies der Gesamtlänge von distaler und proximaler Elektrode). Dieser Abstand hat sich als besonders geeignet für die Gewebeverödung mit Hilfe eines hochfrequenten Wechselstroms erwiesen.

Der Durchmesser des Hochfrequenzkatheters, insbesondere der Außendurchmesser des Schaftrohres sowie der Außendurchmesser der proximalen Elektrode und der größte Durchmesser der Kopfelektrode sind vorzugsweise einander annähernd gleich. Die Kopfelektrode besitzt dabei vorzugsweise ihren größten Durchmesser am proximalen Ende ihrer nach außen gerichteten Umfangswandung. Zu ihrem distalen Ende hin ist die Kopfelektrode beispielsweise halbkugelförmig geformt. Alternativ dazu kann sie einen Trokarschliff besitzen oder kegelförmig oder keilförmig angespitzt sein.

Der größte Durchmesser der Kopfelektrode sowie der Durchmesser der proximalen Elektrode und des übrigen Hochfrequenzkatheters beträgt vorzugsweise weniger als 3,5 mm.

Im Hinblick auf bevorzugte Anwendungen beträgt die Länge des Schaftrohres zwischen 300 und 3000 mm. Entsprechend lang ist auch das durchgehende Zugelement zu messen.

Eine derartige Applikationsvorrichtung eignet sich für bekannte Anwendungen und erschließt darüber hinaus neue Therapieverfahren und Anwendungsgebiete.

### Endoluminaler Einsatz der Vorrichtung:

Zum endoluminalen Einsatz wird der Hochfrequenzkatheter der Applikationsvorrichtung in das Lumen eines Hohlorgans, insbesondere Gefäßes eingeführt. Anschließend wird eine hochfrequente Wechselspannung an den Elektroden angelegt. Durch Vorschieben und Zurückziehen bzw. Hin- und Herbewegen oder einer Drehbewegung um die eigene Achse kann die Position der Elektroden im Hohlorgan verändert werden.

Für den endoluminalen Einsatz ist die Kopfelektrode abgerundet und ist in ihrer Längserstreckung kürzer als die proximale Elektrode. Der Durchmesser des Hochfrequenzkatheters einer Applikationsvorrichtung zum endoluminalen Einsatz ist dem Durchmesser des zu behandelnden Hohlorgan bzw. Gefäßes anzupassen. Bei endoluminalem Einsatz wird das zu behandelnde Hohlorgan, insbesondere die Gefäßwand, von Strom durchflossen, erwärmt sich und koaguliert. Das Hohlorgan bzw. das Gefäß reduziert in der Folge seinen Innendurchmesser oder wird völlig verschlossen.

### Behandlungsablauf am Beispiel varikoser Venen:

Ein zu behandelndes Blutgefäß, vorzugsweise die Vena Saphena Magna, wird zunächst vorzugsweise in der Nähe des Fußknöchels geöffnet. Anschließend wird der Hochfrequenzkatheter mit seinem distalen Ende voran in die geöffnete Vene eingeführt und bis zum Ende der Vene vorgeschoben. Im Falle der Vena Spahena Magna ist dies der Übergang zur Vena Femoralis. Wahlweise kann auch die Vena Spahena Magna von der Vena Femoralis getrennt werden (Krossektomie) und die Elektrode vorgeschoben werden, bis sie an dieser Öffnung sicht bar wird. Dabei wird noch keine hochfrequente, zu einer Koagulation führende Wechselspannung an die Elektrode oder die Elektroden des Hochfrequenzkatheters angelegt. Wird keine Krossektomie durchgeführt, wird die Position des Elektrodenkopfes am saphenofemoralen Übergang vorzugsweise mit songraphischer Bildgebung kontrolliert.

Nachdem das distale Ende des Hochfrequenzkatheters mit der Kopfelektrode richtig positioniert ist, kann an die für die Behandlung vorgesehene Elektrode eine hochfrequente Wechselspannung angelegt werden, die eine Koagulation bewirkt. In einer monopolaren Anordnung ist die für die Behandlung vorgesehene Elektrode die Kopfelektrode am distalen Ende des Hochfrequenzkatheters. Eine Gegenelektrode wird als großflächige neutrale Elektrode zuvor am Körper des Patienten angelegt. Falls gemäß einer bevorzugten Variante ein bipolarer Hochfrequenzkatheter verwendet wird, wird die hochfrequente Wechselspannung zwischen der Kopfelektrode und der zugeordneten proximalen Ringelektrode angelegt.

Um das Blutgefäß auf der gewünschten Länge per Koagulation zu verengen, wird dann der Hochfrequenzkatheter langsam in proximale Richtung zurückgezogen. Die Arbeitsgeschwindigkeit wird dabei an die Geometrie des zu behandelnden Blutgefäßes sowie an die angelegte, hochfrequente Wechselspannung angepasst.

Zur Steigerung des Therapieeffektes kann vor dem Anlegen der hochfrequenten Wechselspannung das sich in der Vene befindende Blut mit einer Manschette über die gesamte Länge der Vene herausgedrückt werden.

Um während des Koagulationsvorganges (Rückzugsverfahren) in etwa die Position des Elektrodenkopfes am distalen Ende des Hochfrequenzkatheters abschätzen zu können, ist es vorteilhaft, wenn parallel zu dem Hochfrequenzkatheter vom Verbindungselement der Applikationsvorrichtung ausgehend eine Schnur so gespannt wird, dass sich das Ende der Schnur oder eine markierte Stelle der Schnur außerhalb des Körpers des Patienten etwa gleichauf mit der Kopfelektrode innerhalb des Patienten befindet. Auf diese Weise lässt sich der Hochfrequenzkatheter besonders gefühlvoll und mit gleichmäßiger Geschwindigkeit in proximale Richtung zurückziehen. Weitere Möglichkeiten der Positionskontrolle sind sonographische Bildgebung und Palpation.

Sobald die Kopfelektrode den zu behandelnden Abschnitt eines Blutgefäßes verlässt, werden die Elektroden wieder von der hochfrequenten Wechselspannung getrennt und der Hochfrequenzkatheter kann ganz aus dem Körper des Patienten zurückgezogen werden.

Falls die Applikationsvorrichtung an ein entsprechendes Steuergerät angeschlossen ist, kann die hochfrequente Wechselspannung während der Koagulation an die jeweiligen Anforderungen adaptiert werden. Falls das Steuergerät so beschaffen ist, dass es beispielsweise ein von der Impedanz zwischen der Kopfelektrode und der Gegenelektrode abhängiges akustisches oder optisches Signal abgibt, kann sowohl die Geschwindigkeit des Zurückziehens des Hochfrequenzkatheters als auch die Größe der hochfrequenten Wechselspannung besonders leicht den jeweiligen Erfordernissen angepasst werden.

### Behandlungsablauf am Beispiel des Eileiters:

Ein weiteres Einsatzgebiet einer Applikationsvorrichtung mit endoluminaler Ausprägung liegt in der Verengung bzw. Verödung eines Eileiters zu Sterilisationszwecken. Mittels eines Hysteroskops (Endoskop für die Gynäkologie) wird die Elektrode von der Gebärmutter aus in den zu verschließenden Eileiter eingeführt. Der weitere Verlauf gleicht dem der Verengung von Venen (s.o.): Nach korrekter Positionierung der Elektrode innerhalb des Eileiters wird Hochfrequenzstrom abgegeben und die Elektrode eine definierte Strecke zurückgezogen, so das sich der koagulierte Bereich bezüglich des Durchmessers zusammenzieht und dadurch verschließt.

### Interstitiell-endoskopischer Einsatz:

Zum interstitiell-endoskopischen Einsatz wird der Hochfrequenzkatheter der Applikationsvorrichtung in einen Arbeitskanal eines Endoskops eingeführt und bis zum Behandlungsort vorgeschoben, um dort in das zu behandelnde Gewebe einzudringen und bei Anlegen einer Wechselspannung das Gewebe infolge der Koagulation zu zerstören. Der Einsatz kann beispielsweise unter sonographischer Kontrolle stattfinden.

Zum interstitiell-endoskopischen Einsatz weist die Kopfelektrode vorzugsweise einen Kegel- oder Trokarschliff auf. Weiterhin ist vorzugsweise die Elektrodenoberfläche zumindest bereichsweise so beschaffen, dass die Reflexion von Ultraschall verstärkt wird, um eine sonographische Kontrolle der Applikation zu erleichtern. Schließlich ist die Applikationsvorrichtung in interstitiell-endoskopischer Verwendung vorzugsweise mit einem Führungsschlauch bzw. Führungssystem ausgebildet. Für den interstitiell-endoskopischen Einsatz muss der Durchmesser des Hochfrequenzkatheters auf den Durchmesser des Arbeitskanals des Endoskops abgestimmt sein.

### Behandlungsablauf am Beispiel von Pankreastumoren:

Ein Einsatzgebiet einer Applikationsvorrichtung mit interstitiell-endoskopischer Ausprägung ist die Behandlung von Pankreastumoren (Pankreaskarzinomen). Der röhrenförmige Hochfrequenzkatheter der Applikationsvorrichtung wird dazu über einen Arbeitskanal eines Gastroskopes (Endoskop für den Gastrointestinalbereich) in den Magen eingeführt und auf die Magenwand über dem Pankreas aufgesetzt. Unter sonographischer Kontrolle (endoskopischer Ultraschall) wird der Pankreastumor punktiert und das Tumorgewebe wird durch Koagulation zerstört.

### Behandlungsablauf am Beispiel von Bronchialtumoren:

Ein weiteres Einsatzgebiet einer Applikationsvorrichtung mit interstitiell-endoskopischer Ausprägung ist die Behandlung von Bronchialtumoren. Dazu wird der Hochfrequenzkatheter durch ein Bronchoskop (Endoskop für den Bronchialbereich) in den betroffenen Bronchus eingeführt und mit dem distalen Ende vor dem Tumor platziert. Der Tumor - der z. B. den Bronchus obstruieren kann und dadurch die Atemluftversorgung des sich anschließenden Bereichs verhindert - wird ein oder mehrfach mit der in einen Arbeitskanal des Bronchoskops eingeführten Spitze des röhrenförmigen Hochfrequenzkatheters punktiert, wobei jeweils eine hochfrequente Wechselspannung angelegt wird. Das Gewebe des Bronchialtumors koaguliert. Die durch die Koagulation hervorgerufene Volumenreduktion des Tumorgewebes soll insbesondere zu einer Querschnittsvergrößerung unter Beseitigung der Obstruktion des Bronchus führen.

### Behandlungsablauf am Beispiel von Tumoren am Gallengang:

Ein weiteres Einsatzgebiet einer Applikationsvorrichtung mit interstitiell-endoskopischer Ausprägung ist die Behandlung obstruierender Tumore im oder am Gallengang (ductus choledochus). Die einzelnen Schritte der Behandlung sind mit einem Gastroskop analog der Behandlung von obstruierenden Bronchialtumoren durchzuführen. Der Hochfrequenzkatheter wird durch ein Gastroskop (Endoskop für den Gastrointestinalbereich) in den Gallengang eingeführt und mit dem distalen Ende vor dem Tumor platziert. Der Tumor - der den Gallengang obstruiert wird ein oder mehrfach mit der in einen Arbeitskanal des Gastroskops eingeführten Spitze des röhrenförmigen Hochfrequenzkatheters punktiert, wobei jeweils eine hochfrequente Wechselspannung angelegt wird, um das Tumorgewebe zu koagulieren. Anschließen kann an dieser Stelle ein Stent (Röhrenförmiges Element, mit dem ein Lumen gegen Obstruktion gesichert werden kann) in den Gallengang eingesetzt werden, wodurch ein sofortiger Abfluss der Gallenflüssigkeit ermöglicht wird.

### Behandlungsablauf am Beispiel der gastroösophagealen Reflux-Krankheit:

Schließlich eignet sich die Applikationsvorrichtung bei interstitiell-endoskopischer Ausprägung der Applikationsvorrichtung zur Behandlung von gastroösophagealen Reflux-Krankheit (GERD: gastroesophageal reflux disease). Hierzu wird der Hochfrequenzkatheter der Applikationsvorrichtung über ein Endoskop zum Mageneingang (Sphinkter) geführt. Das distale Ende des Endoskops wird auf Höhe des Sphinkters um ca. 30 - 90° abgewinkelt, die Elektrode vorgeschoben und in das Sphinktergewebe eingestochen. Danach wird Hochfrequenzstrom abgegeben, um das Gewebe zu koagulieren. Der dadurch indizierte Vernarbungsprozess führt zu einer Verengung des Mageneinganges und der Rückfluss von Magensäure in die Speiseröhre wird verhindert oder vermindert.

Die Erfindung wird nachfolgend anhand eines Ausführungsbeispiels und der dazugehörigen Zeichnungen näher erläutert. Es zeigen:
- Figuren 1a und 1b:: eine erfindungsgemäße Applikationsvorrichtung mit einer vergrößerten Darstellung ihres distalen Endes,
- Figur 2:: eine Kopfelektrode mit einem Zugelement, das gleichzeitig als elektrische Zuleitung dient,
- Figur 3a und 3b:: eine beispielhafte, vergrößerte schematische Schnittdarstellung eines geschlossenen Flüssigkeitskreislaufs einer Ausführungsvariante des distalen Endes der erfindungsgemäßen Applikationsvorrichtung und
- Figuren 4a und 4b:: eine beispielhafte, vergrößerte schematische Schnittdarstellung einer offenen Flüssigkeitsspülung einer Ausführungsvariante des distalen Endes der erfindungsgemäßen Applikationsvorrichtung.

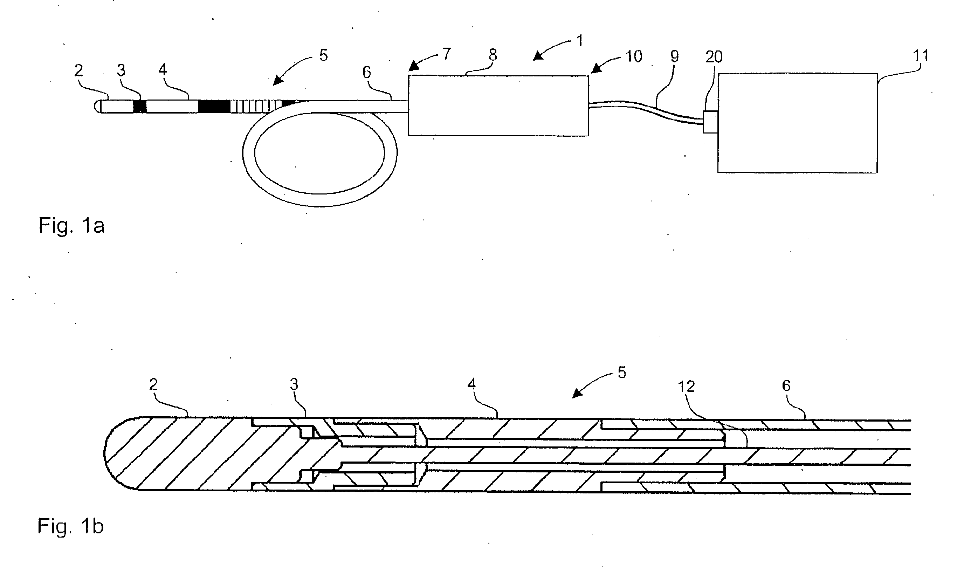

Fig. 1a zeigt eine biegeweiche Applikationsvorrichtung 1, die zur Hochfrequenztherapie von biologischem Gewebe geeignet ist. Die Applikationsvorrichtung 1 umfasst ein Verbindungselement 8, in dessen Inneren sich die elektrische und mechanische Schnittstelle zwischen dem Kabel 9 und den Leitungen innerhalb des Hochfrequenzkatheters 5 befindet.

An seinem proximalen Ende 10 weist das Verbindungselement 8 eine Durchführung auf, durch die ein Kabel 9 geführt ist. Das Kabel 9 ist über den Anschluss 20 mit einer Versorger- und Steuereinheit 11 verbunden, mit der eine hochfrequente Wechselspannung in an sich bekannter Weise erzeugt werden kann.

Am distalen Ende 7 des Verbindungselements 8 ist ein biegeweicher, röhrenförmiger Hochfrequenzkatheter 5 durch eine geeignete Öffnung in das Innere des Verbindungselements 8 geführt und dort befestigt.

Der Hochfrequenzkatheter 5 umfasst ein Schaftrohr 6 mit einem durchgehenden Lumen. Die Hülle des Schaftrohres 6 ist aus einem elastischen Material, z. B. einem Polymer, insbesondere PEEK, geformt.

Weiterhin umfasst der Hochfrequenzkatheter 5 - ausgehend vom Verbindungselement 8 in distaler Richtung nacheinander angeordnet - eine proximale Elektrode 4, einen Isolator 3 und eine am distalen Ende des Hochfrequenzkatheters 5 angeordnete Kopfelektrode 2.

Die Kopfelektrode 2 hat im abgebildeten Ausführungsbeispiel einen abgerundeten Kopf und eignet sich daher insbesondere für den endoluminalen Einsatz. Für interstitiell-endoskopische Verwendungszwecke kann die Kopfelektrode 2 an ihrem distalen Ende angespitzt sein und besitzt vorzugsweise einen Trokarschliff oder ist keilförmig oder kegelförmig angespitzt.

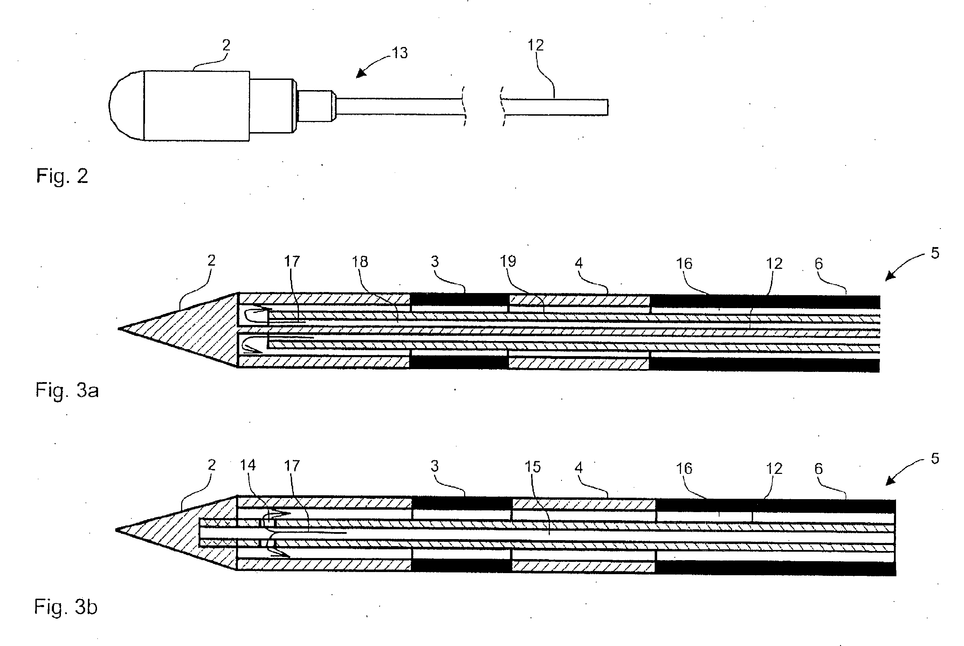

Fig. 1b zeigt eine vergrößerte Darstellung des distalen Endes der erfindungsgemäßen Applikationsvorrichtung 1. Das Zugelement 12 für die Kopfelektrode 2 ist mittig durch das Schaftrohr 6 und, elektrisch gegenüber der proximalen Elektrode 4 isoliert, bis zum proximalen Ende 13 (siehe auch Fig. 2) der Kopfelektrode 2 geführt. Das Zugelement 12 ist als Metalldraht ausgeführt, der vorzugsweise aus nichtrostendem V2A-Stahl besteht. Zur Erhöhung der Flexibilität kann dieser als geflochtenes Metallseil ausgeführt sein. Zum Zwecke der elektrischen Isolierung kann der Draht teilweise von einem nichtleitendem Material ummantelt sein. Das Zugelement 12 ist mit seinem distalen Ende am proximalen Ende 13 der Kopfelektrode 2 fest verbunden, vorzugsweise angeschweißt. Auch die Kopfelektrode 2 besteht zumindest im Bereich ihres proximalen Endes 13 aus V2A-Stahl. Das Zugelement 12 besitzt eine Zugfestigkeit von mindestens 1000 N/mm². Im beschriebenen Ausführungsbeispiel hat das Zugelement 12 der Kopfelektrode 2 einen Durchmesser von 0,4 mm. Proximal ist das Zugelement 12 mit dem Verbindungselement 8 mechanisch stabil und mit dem Kabel 9 innerhalb des Verbindungselements 8 elektrisch leitend verbunden.

Durch das Lumen des Schaftrohres 6 ist eine - hier nicht näher erläuterte - elektrische Zuleitung zur proximalen Elektrode 4 geführt, die mit dem Kabel 9 innerhalb des Verbindungselements 8 elektrisch leitend verbunden ist. Die elektrische Zuleitung für die proximale Elektrode 4 besteht beispielsweise aus Kupfer und ist mit einem Isolatormaterial ummantelt.

Fig. 2 zeigt die Kopfelektrode 2 mit einem Zugelement 12. Das Zugelement 12 ist mit seinem distalen Ende am proximalen Ende 13 der Kopfelektrode 2 fest verbunden, vorzugsweise angeschweißt. Auch die Kopfelektrode 2 besteht zumindest im Bereich ihres proximalen Endes 13 aus V2A-Stahl.

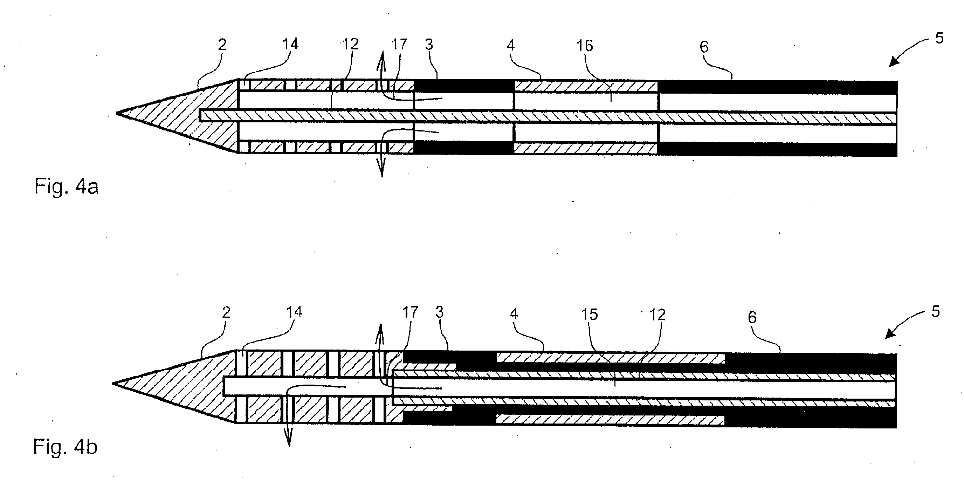

Der Hochfrequenzkatheter 5 kann zur Steigerung der Koagulationseffizienz auch mit einer Elektrodentemperierung versehen werden. Hierzu wird in einer Ausführungsvariante über dem Zugelement 12 ein Spülrohr (hohl) angeordnet. Weiterhin kann in einer Ausführungsvariante das Zugelement 12 nicht als Draht (Vollmaterial), sondern als Rohr (hohl) ausgeführt sein. Im folgenden sollen diese Ausführungsvarianten anhand von Fig. 3a, Fig. 3b, Fig. 4a und Fig. 4b näher erläutert werden.

Fig. 3a und Fig. 3b zeigen eine beispielhafte, vergrößerte schematische Schnittdarstellung eines geschlossenen Flüssigkeitskreislaufs (Gegenstromprinzip) des distalen Endes der erfindungsgemäßen Applikationsvorrichtung.

Gemäß Fig. 3a ist über dem Zugelement 12 ein Spülrohr 19 angeordnet. Durch das Innenlumen 18 des Spülrohrs 19 wird Flüssigkeit 17 zum distalen Ende des Hochfrequenzkatheters 5 gepumpt, gelangt durch das offene distale Ende des Spülrohrs 19 in den Raum 16 zwischen Spülrohr 19 und der Kopfelektrode 2, dem Isolator 3, der proximalen Elektrode 4, dem Schaftrohr 6 und fließt zurück zum proximalen Ende des Hochfrequenzkatheters 5. Das Spülrohr 19 besteht vorzugsweise aus einem nichtleitenden biegeweichen Material.

Fig. 3b zeigt eine Ausführungsvariante in der das Zugelement 12 nicht als Draht (Vollmaterial), sondern als Rohr (hohl) ausgeführt ist. Durch das Innenlumen 15 des Zugelements 12 wird Flüssigkeit 17 zum distalen Ende des Hochfrequenzkatheters 5 gepumpt, gelangt durch die Querbohrungen 14 in den Raum 16 zwischen dem Zugelement 12 und der Kopfelektrode 2, dem Isolator 3, der proximalen Elektrode 4, dem Schaftrohr 6 und fließt zurück zum proximalen Ende des Hochfrequenzkatheters 5. In dieser Ausführungsvariante besteht das Zugelement 12 vorzugsweise aus einer leitenden Metalllegierung wie NiTiNol (Legierung bestehend aus Nickel und Titan) oder CuAlZn (Legierung bestehend aus Kupfer, Aluminium und Zink) oder AuCd (Legierung bestehend aus Gold und Cadmium) oder FePt (Legierung bestehend aus Eisen und Platin).

Bei Hochfrequenzkathetern 5 mit geringem Außendurchmesser kann eine offene Spülung realisiert werden. Dies kann in zwei Varianten (gemäß Fig. 4a und 4b) geschehen:
Fig 4a und 4b zeigen eine beispielhafte, vergrößerte schematische Schnittdarstellung einer offenen Flüssigkeitsspülung einer Ausführungsvariante des distalen Endes der erfindungsgemäßen Applikationsvorrichtung.

Gemäß Fig. 4a ist das Zugelement 12 zur Kopfelektrode 2 als Draht ausgeführt. Die Flüssigkeit 17 wird durch den Raum 16 zwischen dem Zugelement 12, dem Schaftrohr 6, der proximalen Elektrode 4, dem Isolator 3 und der Kopfelektrode 2 gepumpt und tritt durch Bohrungen 14 oder Schlitze 14 in der Kopfelektrode 2 aus. In einer weiteren Ausführungsvariante können sich auch Bohrungen 14 oder Schlitze 14 in der proximalen Elektrode 4 befinden.

Gemäß Fig. 4b ist das Zugelement 12 zur Kopfelektrode 2 als biegeweiches Rohr ausgeführt. Die Flüssigkeit 17 wird durch das Innenlumen 15 des Zugelements 12 zum distalen Ende gepumpt und tritt durch Bohrungen 14 oder Schlitze 14 in der Kopfelektrode 2 aus. In dieser Ausführungsvariante besteht das Zugelement 12 vorzugsweise aus elastischen und leitenden Metalllegierungen wie NiTiNol (Legierung bestehend aus Nickel und Titan) oder CuAlZn (Legierung bestehend aus Kupfer, Aluminium und Zink) oder AuCd (Legierung bestehend aus Gold und Cadmium) oder FePt (Legierung bestehend aus Eisen und Platin).

## Patentansprüche

1. Biegeweiche Applikationsvorrichtung (1) für eine endoluminale oder eine interstitiell-endoskopische Anwendung zur Hochfrequenztherapie von biologischem Gewebe oder Hohlorganen, umfassend einen röhrenförmigen Hochfrequenzkatheter (5) mit
- einem biegeweichen, zumindest ein durchgehendes Lumen aufweisenden Schaftrohr (6),
- einer am distalen Ende des Hochfrequenzkatheters (5) angeordneten Kopfelektrode (2),
- einer elektrischen Leitung (9) mit einem Anschluss (20) für einen Hochfrequenzgenerator (11), und
- einem Verbindungselement (8) zwischen dem Schaftrohr (6) und der Leitung (9),
wobei sich in dem Lumen zwischen der Kopfelektrode (2) und dem Verbindungselement (8) ein Zugelement (12) erstreckt, das mit der Kopfelektrode (2) einerseits und dem Verbindungselement (8) andererseits derart fest verbunden ist und dessen Querschnittsfläche und Zugfestigkeit so bemessen sind, dass sämtliche während einer Behandlung auftretenden äußeren, auf den Hochfrequenzkatheter (5) einwirkenden Kräfte über die Kopfelektrode (2) und das Zugelement (12) in das Verbindungselement (8) übertragen werden können, wobei das Zugelement (12) elektrisch leitend und sowohl mit der Kopfelektrode (2) als auch mit dem Anschluss (20) für einen Hochfrequenzgenerator elektrisch leitend verbunden ist und
wobei das Zugelement (12) und die Verbindung zu der Kopfelektrode (2) eine maximale Zugkraft im Bereich von 70 bis 300 N aufweist.

2. Applikationsvorrichtung nach Anspruch 1, **dadurch gekennzeichnet, dass** der Hochfrequenzkatheter (5) eine elektrisch isolierende Hülle aufweist, die das Lumen einschließt.

3. Applikationsvorrichtung nach Anspruch 1 oder 2, **dadurch gekennzeichnet, dass** das Zugelement (12) ganz oder in Teilen aus nichtrostendem Stahl oder aus einer leitenden Metalllegierung besteht.

4. Applikationsvorrichtung nach einem der Ansprüche 1 bis 3, **dadurch gekennzeichnet, dass** das Zugelement ein innen hohles Rohr (15) ist.

5. Applikationsvorrichtung nach einem der Ansprüche 1 bis 4, **dadurch gekennzeichnet, dass** das Zugelement (12) einen Durchmesser im Bereich von 0,2 bis 0,8 mm, insbesondere von ca. 0,4 mm hat.

6. Applikationsvorrichtung nach einem der Ansprüche 1 bis 3 oder 5, **dadurch gekennzeichnet, dass** das Zugelement (12) ein Draht ist.

7. Applikationsvorrichtung nach Anspruch 6, **dadurch gekennzeichnet, dass** das Zugelement (12) ein geflochtener Draht ist, der eine Vielzahl von einzelnen Filamenten besitzt.

8. Applikationsvorrichtung nach Anspruch 4, **dadurch gekennzeichnet, dass** das Zugelement (12) ein innen hohles Rohr ist, welches aus einem flüssigkeitsdichten Kunststoff besteht, der von einem elektrisch leitenden Drahtgeflecht ummantelt ist oder in den ein elektrisch leitendes Drahtgeflecht eingearbeitet ist.

9. Applikationsvorrichtung nach einem der Ansprüche 1 bis 8, **dadurch gekennzeichnet, dass** die Kopfelektrode (2) zumindest in einem Anschlussbereich des Zugelements (12) aus nichtrostendem Stahl oder aus einer leitenden Metalllegierung besteht.

10. Applikationsvorrichtung nach einem der Ansprüche 1 bis 9, **dadurch gekennzeichnet, dass** das Schaftrohr (6) einen Außendurchmesser aufweist, der dem Außendurchmesser der Kopfelektrode (2) an deren proximalen Ende näherungsweise entspricht.

11. Applikationsvorrichtung nach einem der Ansprüche 1 bis 10, **dadurch gekennzeichnet, dass** der Hochfrequenzkatheter (5) eine zweite, gegenüber der Kopfelektrode (2) elektrisch isolierte, proximale Elektrode (4) aufweist, die in der Nähe des distalen Endes des Hochfrequenzkatheters (5) proximal der Kopfelektrode (2) angeordnet und über eine zweite elektrisch leitende Zuleitung mit einer zweiten Kontaktstelle in dem Verbindungselement (8) elektrisch leitend verbunden ist.

12. Applikationsvorrichtung nach Anspruch 11, **dadurch gekennzeichnet, dass** der Abstand der proximalen Elektrode (4) von der Kopfelektrode (2) in Längsrichtung des Hochfrequenzkatheters (5) zwischen 5 und 20 % der Gesamtelektrodenlänge, das heißt der Summe der Längen von distaler und proximaler Elektrode, beträgt.

13. Applikationsvorrichtung nach Anspruch 11 oder 12, **dadurch gekennzeichnet, dass** das Schaftrohr (6) einen Außendurchmesser aufweist, der dem Außendurchmesser der proximalen Elektrode (4) und dem größten Durchmesser der Kopfelektrode (2) näherungsweise entspricht.

14. Applikationsvorrichtung nach einem der Ansprüche 1 bis 13, **dadurch gekennzeichnet, dass** der Außendurchmesser kleiner oder gleich 3,5 mm beträgt.

15. Applikationsvorrichtung nach einem der Ansprüche 1 bis 14, **dadurch gekennzeichnet, dass** die Länge des Schaftrohres (6) zwischen 300 und 3000 mm beträgt.

16. Applikationsvorrichtung nach Anspruch 15, **dadurch gekennzeichnet, dass** die Länge des Zugelementes (12) zwischen 300 und 3000 mm beträgt.

17. Applikationsvorrichtung nach Anspruch 10 oder 13, **dadurch gekennzeichnet, dass** der Außendurchmesser der Kopfelektrode (2) an deren proximalen Ende der größte Durchmesser der Kopfelektrode (2) ist.

18. Applikationsvorrichtung nach einem der Ansprüche 1 bis 17, **dadurch gekennzeichnet, dass** die Applikationsvorrichtung (1) zum endoluminalen oder interstitiell-endoskopischen Einsatz ausgebildet ist.

19. Applikationsvorrichtung nach einem der Ansprüche 1 bis 18, **dadurch gekennzeichnet, dass** die Kopfelektrode (2) am distalen Ende nach Art einer Trokarspitze geformt ist.

20. Applikationsvorrichtung nach einem der Ansprüche 1 bis 18, **dadurch gekennzeichnet, dass** die Kopfelektrode (2) am distalen Ende halbkugelförmig ist.

21. Applikationsvorrichtung nach einem der Ansprüche 1 bis 18, **dadurch gekennzeichnet, dass** die Kopfelektrode (2) am distalen Ende kegelförmig ausläuft.

22. Applikationsvorrichtung nach Anspruch 5 und einem der Ansprüche 8 bis 31, **dadurch gekennzeichnet, dass** ein Innenlumen (15) des Zugelements (12) sowie ein entlang des Zugelements (12) verlaufender Hohlraum (16) des Hochfrequenzkatheters (5) im Bereich des distalen Endes des Hochfrequenzkatheters (5) miteinander fluidverbunden sind und ein geschlossenes Flüssigkeitsleitsystem bilden.

23. Applikationsvorrichtung nach Anspruch 22, **dadurch gekennzeichnet, dass** das Innenlumen (15) des Zugelements (12) im distalen Bereich mit mindestens einer Querbohrung (14) versehen ist, durch die Flüssigkeit in den Hohlraum (16) zwischen dem Zugelement (12) und einem Schaftrohr (6) gelangen und zum proximalen Ende des Hochfrequenzkatheters (5) zurückfließen kann.

24. Applikationsvorrichtung nach Anspruch 6 oder 7 und einem der Ansprüche 10 bis 22, **dadurch gekennzeichnet, dass** über dem Zugelement (12) ein nichtleitendes biegeweiches Spülrohr (19) angeordnet sowie ein entlang des Spülrohrs (19) verlaufender Hohlraum (16) des Hochfrequenzkatheters (5) im Bereich des distalen Endes des Hochfrequenzkatheters (5) miteinander fluidverbunden sind und ein geschlossenes Flüssigkeitsleitsystem bilden.

25. Applikationsvorrichtung nach Anspruch 24, **dadurch gekennzeichnet, dass** das Innenlumen (18) des Spülrohrs (19) im distalen Bereich eine Öffnung besitzt, durch die Flüssigkeit in den Hohlraum (16) zwischen dem Zugelement (12) und dem Schaftrohr (6) gelangen und zum proximalen Ende des Hochfrequenzkatheters (5) zurückfließen kann.

26. Applikationsvorrichtung nach einem der Ansprüche 1 bis 21, **dadurch gekennzeichnet, dass** am distalen Ende des Hochfrequenzkatheters (5) oder in dessen Nähe mindestens eine Öffnung (14) vorgesehen ist, die von einem sich vom proximalen Ende des Schaftrohres (6) bis zu der mindestens einen Öffnung (14) erstreckenden Hohlraum (16) zwischen dem Zugelement (12) und dem Schaftrohr (6) nach außen führen und es erlauben, Flüssigkeit durch den Hohlraum (16) zu der mindestens einen Öffnung (14) zu leiten und dort austreten zu lassen.

27. Applikationsvorrichtung nach Anspruch 4 und einem der Ansprüche 8 bis 21, **dadurch gekennzeichnet, dass** am distalen Ende des Hochfrequenzkatheters (5) oder in dessen Nähe mindestens eine Öffnung (14) vorgesehen ist, die von dem Innenlumen (15) des Zugelements (12) nach außen führen und es erlauben, Flüssigkeit zu der mindestens einen Öffnung (14) zu leiten und dort austreten zu lassen.

28. Applikationsvorrichtung nach Anspruch 26 oder 27, **dadurch gekennzeichnet, dass** die Öffnungen Bohrungen oder Schlitze (14) in den Elektroden sind.

## Claims

1. Flexible application device (1) for an endoluminal or an interstitial-endoscopic application for high-frequency therapy of biological tissue or hollow organs, comprising a tubular high-frequency catheter (5) with
- a flexible shaft tube (6), comprising at least one continuous lumen,
- a head electrode (2) arranged at the distal end of the high-frequency catheter (5),
- an electric cable (9) with a connector (20) for a high-frequency generator (11), and
- a connection element (8) between the shaft tube (6) and the cable (9),
wherein a traction element (12) extends in the lumen between the head electrode (2) and the connection element (8), which is firmly connected with the head electrode (2), on the one hand, and with the connecting element (8), on the other hand, such, and the cross-section area and tensile strength of which are configured such, that all during a treatment occurring outer forces acting on the high-frequency catheter (5) can be transmitted by means of the head electrode (2) and the traction element (12) to the connecting element (8), wherein the traction element (12) is electrically conductive and electrically connected both with the head electrode (2) and with the connector (20) for a high-frequency generator, and
wherein the traction element (12) and the connection to the head electrode (2) has a maximum tensile force in the range of 70 to 300 N.

2. Application device according to claim 1, **characterized in that** the high-frequency catheter (5) has an electrically isolating sheath that encloses the lumen.

3. Application device according to claim 1 or 2, **characterized in that** the traction element (12) wholly or in parts consists of stainless steel or of a conductive metal alloy.

4. Application device according to one of claims 1 to 3, **characterized in that** the traction element is an inside hollow tube (15).

5. Application device according to one of claims 1 to 4, **characterized in that** the traction element (12) has a diameter in the range of 0.2 to 0.8 mm, particularly of 0.4 mm.

6. Application device according to one of claims 1 to 3 or 5, **characterized in that** the traction element (12) is a wire.

7. Application device according to claim 6, **characterized in that** the traction element (12) is a braided wire, which has a plurality of single filaments.

8. Application device according to claim 4, **characterized in that** the traction element (12) is an inside hollow tube, which consists of a liquid-tight plastic, which is coated by an electrically conductive wire mesh or into which an electrically conductive wire mesh is incorporated.

9. Application device according to one of claims 1 to 8, **characterized in that** the head electrode (2) consists, at least at one terminal portion of the traction element (12), of stainless steel or of a conductive metal alloy.

10. Application device according to one of claims 1 to 9, **characterized in that** the shaft tube (6) has an outer diameter, which approximately corresponds to the outer diameter of the head electrode (2) at its proximal end.

11. Application device according to one of claims 1 to 10, **characterized in that** the high-frequency catheter (5) has a second, vis-à-vis the head electrode (2) electrically isolated proximal electrode (4), which is arranged in the proximity of the distal end of the high-frequency catheter (5) proximal of the head electrode (2) and which is via a second electrically conductive supply line electrically conductive connected to a second contact point in the connecting element (8).

12. Application device according to claim 11, **characterized in that** the distance of the proximal electrode (4) to the head electrode (2) in longitudinal direction of the high-frequency catheter (5) is between 5 and 20 % of the total electrode length, i.e. the sum of lengths of distal and proximal electrode.

13. Application device according to claim 11 or 12, **characterized in that** the shaft tube (6) has an outer diameter, which approximately corresponds to the outer diameter of the proximal electrode (4) and the greatest diameter of the head electrode (2).

14. Application device according to one of claims 1 to 13, **characterized in that** the outer diameter is smaller than or equal 3.5 mm.

15. Application device according to one of claims 1 to 14, **characterized in that** the length of the shaft tube (6) is between 300 and 3000 mm.

16. Application device according to claim 15, **characterized in that** the length of the traction element (12) is between 300 and 3000 mm.

17. Application device according to claim 10 or 13, **characterized in that** the outer diameter of the head electrode (2) at its proximal end is the greatest diameter of the head electrode (2).

18. Application device according to one of claims 1 to 17, **characterized in that** the application device (1) is configured for endoluminal or interstitial-endoscopic application.

19. Application device according to one of claims 1 to 18, **characterized in that** the head electrode (2) is formed at its distal end like a trocar tip.

20. Application device according to one of claims 1 to 18, **characterized in that** the head electrode (2) is hemispherical at the distal end.

21. Application device according to one of claims 1 to 18, **characterized in that** the head electrode (2) tapers off conical at the distal end.

22. Application device according to claim 5 and one of claims 8 to 31, **characterized in that** an inner lumen (15) of the traction element (12) and a hollow space (16) of the high-frequency catheter (5) extending along the traction element (12) are fluidly connected to one another in the portion of the distal end of the high-frequency catheter (5) and form a closed fluid control system.

23. Application device according to claim 22, **characterized in that** the inner lumen (15) of the traction element (12) has at least one cross hole (14) at the distal portion, through which liquid can reach into the hollow space (16) between the traction element (12) and a shaft tube (6) and flow back to the proximal end of the high-frequency catheter (5).

24. Application device according to claim 6 or 7 and one of claims 10 to 22, **characterized in that** a non-conductive flexible wash pipe (19) arranged above the traction element (12) and a hollow space (16) of the high-frequency catheter (5) extending along the wash pipe (19) are in the portion of the distal end of the high-frequency catheter (5) fluidly connected to one another and form a closed fluid control system.

25. Application device according to claim 24, **characterized in that** the inner lumen (18) of the wash pipe (19) has an opening at the distal portion, through which fluid can reach into the hollow space (16) between the traction element (12) and the shaft tube (6) and can flow back to the proximal end of the high-frequency catheter (5).

26. Application device according to one of claims 1 to 21, **characterized in that**, at the distal end of the high-frequency catheter (5) or at its proximity, at least one opening (14) is provided, which leads from a hollow space (16) extending from the proximal end of the shaft tube (6) to the at least one opening (14) between the traction element (12) and the shaft tube (6) outwardly and which allows fluid to be conveyed through the hollow space (16) to the at least one opening (14) and to be discharged there.

27. Application device according to claim 4 and one of claims 8 to 21, **characterized in that**, at the distal end of the high-frequency catheter (5) or at its proximity, at least one opening (14) is provided, which leads from the inner lumen (15) of the traction element (12) outwardly and allows fluid to be conveyed to the at least one opening (14) and to be discharged there.

28. Application device according to claim 26 or 27, **characterized in that** the openings are holes or slots (14) in the electrodes.

## Revendications

1. Dispositif d'application souple (1) prévu pour une utilisation endoluminale ou endoscopique interstitielle destiné à la thérapie à haute fréquence des tissus biologiques ou organes creux, comprenant un cathéter haute fréquence (5) avec
- un tube (6) souple, qui présente au moins une lumière constante,
- une électrode principale (2) à l'extrémité distale du cathéter haute fréquence (5),
- un conducteur électrique (9) équipé d'un raccord (20) pour générateur à haute fréquence (11), et
- un élément de liaison (8) entre la partie tubulaire (6) et le conducteur (9),
où un élément de traction (12) s'étend dans la lumière entre l'électrode principale (2) et l'élément de liaison (8), de telle façon que l'élément de traction (12) est solidement relié d'un côté à l'électrode principale (2) et de l'autre côté à l'élément de liaison (8) et dont la surface de la section et la résistance à la traction sont mesurées de sorte que l'ensemble des forces exercées sur le cathéter haute fréquence (5) dues à des effets extérieurs sur l'électrode principale (2) et l'élément de traction (12), pendant un traitement, puissent être transférées dans l'élément de liaison (8), où l'élément de traction (12) est conducteur et également relié électriquement avec l'électrode principale (2) et le raccord (20) pour générateur à haute fréquence et
où l'élément de traction (12) et la liaison vers l'électrode principale (2) présentent une force de traction maximale dans une plage comprise entre 70 et 300 N.

2. Dispositif d'application selon la revendication 1, **caractérisé en ce que** le cathéter haute fréquence (5) comporte une gaine isolante, d'un point de vue électrique, renfermant la lumière.

3. Dispositif d'application selon la revendication 1 ou 2, **caractérisé en ce que** l'élément de traction (12) se compose, entièrement ou en partie, d'acier inoxydable ou d'un alliage métallique conducteur.

4. Dispositif d'application selon l'une des revendications 1 à 3, **caractérisé en ce que** l'élément de traction est un tube creux (15).

5. Dispositif d'application selon l'une des revendications 1 à 4, **caractérisé en ce que** le diamètre de l'élément de traction (12) est compris entre 0,2 et 0,8 mm, plus particulièrement de 0,4 mm environ.

6. Dispositif d'application selon l'une des revendications 1 à 3 ou 5, **caractérisé en ce que** l'élément de traction (12) est un fil.

7. Dispositif d'application selon la revendication 6, **caractérisé en ce que** l'élément de traction (12) est un fil tressé composé d'une multitude de filaments individuels.

8. Dispositif d'application selon la revendication 4, **caractérisé en ce que** l'élément de traction (12) est un tube creux, en matière plastique étanche aux liquides, entouré d'une toile métallique conductrice ou dans lequel est incorporée une toile métallique conductrice.

9. Dispositif d'application selon l'une des revendications 1 à 8, **caractérisé en ce que** l'électrode principale (2) se compose, au moins dans une zone de raccord de l'élément de traction (12), d'acier inoxydable ou d'un alliage métallique conducteur.

10. Dispositif d'application selon l'une des revendications 1 à 9, **caractérisé en ce que** la partie tubulaire (6) a un diamètre extérieur qui correspond approximativement au diamètre extérieur de l'électrode principale (2) en son extrémité proximale.

11. Dispositif d'application selon l'une des revendications 1 à 10, **caractérisé en ce que** le cathéter haute fréquence (5) comporte une seconde électrode (4) proximale, isolée électriquement vis-à-vis de l'électrode principale (2), placée à proximité de l'extrémité distale du cathéter haute fréquence (5) proximal à l'électrode principal (2) et qui est reliée électriquement via une seconde ligne conductrice à un second point de contact dans l'élément de liaison (8).

12. Dispositif d'application selon la revendication 11, **caractérisé en ce que** l'écart de l'électrode proximale (4) à partir de l'électrode principale (2) dans le sens longitudinal du cathéter haute fréquence (5) se situe entre 5 et 20 % de la longueur totale de l'électrode, c'est-à-dire la somme des longueurs de l'électrode distale et de l'électrode proximale.

13. Dispositif d'application selon la revendication 11 ou 12, **caractérisé en ce que** le diamètre extérieur de la partie tubulaire (6) correspond approximativement au diamètre extérieur de l'électrode proximale (4) et au diamètre le plus large de l'électrode principale (2).

14. Dispositif d'application selon l'une des revendications 1 à 13, **caractérisé en ce que** le diamètre extérieur est inférieur ou égal à 3,5 mm.

15. Dispositif d'application selon l'une des revendications 1 à 14, **caractérisé en ce que** la longueur de la partie tubulaire (6) est comprise entre 300 et 3 000 mm.

16. Dispositif d'application selon la revendication 15, **caractérisé en ce que** la longueur de l'élément de traction (12) est comprise entre 300 et 3 000 mm.

17. Dispositif d'application selon la revendication 10 ou 13, **caractérisé en ce que** le diamètre extérieur de l'électrode principale (2) est le diamètre extérieur le plus large à l'extrémité proximale de l'électrode principale (2).

18. Dispositif d'application selon l'une des revendications 1 à 17, **caractérisé en ce que** le dispositif d'application (1) est prévu pour une utilisation endoluminale ou endoscopique interstitielle.

19. Dispositif d'application selon l'une des revendications 1 à 18, **caractérisé en ce que** l'électrode principale (2) a la forme, en son extrémité distale, d'une pointe de trocart.

20. Dispositif d'application selon l'une des revendications 1 à 18, **caractérisé en ce que** l'électrode principale (2) est de forme hémisphérique en son extrémité distale.

21. Dispositif d'application selon l'une des revendications 1 à 18, **caractérisé en ce que** l'électrode principale (2) est de forme conique en son extrémité distale.

22. Dispositif d'application selon la revendication 5 et l'une des revendications 8 à 31, **caractérisé en ce qu'**une lumière interne (15) de l'élément de traction (12) et un espace vide (16) le long de l'élément de traction (12) du cathéter haute fréquence (5) sont en communication de fluide dans la zone distale du cathéter haute fréquence (5) et forment un système fermé de conduite du fluide.

23. Dispositif d'application selon la revendication 22, **caractérisé en ce que** la lumière interne (15) de l'élément de traction (12) dans la zone distale comporte au moins un alésage transversal (14), permettant au fluide d'accéder à l'espace vide (16) entre l'élément de traction (12) et une partie tubulaire (6) et de refluer vers l'extrémité proximale du cathéter haute fréquence (5)

24. Dispositif d'application selon la revendication 6 ou 7 et l'une des revendications 10 à 22, **caractérisé en ce qu'**un tube de rinçage (19) souple et non conducteur est placé au-dessus de l'élément de traction (12) et un espace vide (16) le long du tube de rinçage (19) du cathéter haute fréquence (5) sont en communication par fluide dans la zone de l'extrémité distale du cathéter haute fréquence (5) et forment un système fermé de conduite du fluide.

25. Dispositif d'application selon la revendication 24, **caractérisé en ce que** la lumière interne (18) du tube de rinçage (19) est pourvue, dans la zone distale, d'un orifice par lequel le fluide peut accéder à l'espace vide (16) entre l'élément de traction (12) et la partie tubulaire (6) et refluer vers l'extrémité proximale du cathéter haute fréquence (5).

26. Dispositif d'application selon l'une des revendications 1 à 21, **caractérisé en ce que**, à l'extrémité distale du cathéter haute fréquence (5) ou dans sa proximité, au moins un orifice (14) est prévu afin de mener le fluide hors de l'espace vide (16) s'étendant de l'extrémité proximale de la partie tubulaire (6) jusqu'à au moins un orifice (14) et situé entre l'élément de traction (12) et la partie tubulaire (6) et permettant de faire circuler le fluide au travers de l'espace vide (16) vers au moins un orifice (14) et d'y évacuer le fluide.

27. Dispositif d'application selon la revendication 4 et l'une des revendications 8 à 21, **caractérisé en ce que**, à l'extrémité distale du cathéter haute fréquence (5) ou dans sa proximité, au moins un orifice (14) est prévu permettant depuis la lumière interne (15) de l'élément de traction (12) de mener vers l'extérieur et de faire circuler le fluide vers au moins un orifice (14) et d'y évacuer le fluide.

28. Dispositif d'application selon la revendication 26 ou 27, **caractérisé en ce que** les orifices sont des perçages ou des fentes (14) situés dans les électrodes.
